# EUROPÄISCHE PATENTSCHRIFT

(11) **EP 3 890 664 B1**
(45) Veröffentlichungstag und Bekanntmachung des Hinweises auf die Patenterteilung: **25.01.2023**
(21) Anmeldenummer: 19817237.1
(22) Anmeldetag: 05.12.2019
(51) Int. Cl.: A61F 5/01, A61F 2/50, F16F 9/19, F16F 9/512, F16F 9/516, F16F 9/30, F16F 9/32

(54) **VORRICHTUNG ZUR STABILISIERUNG VON KÖRPERGELENKEN UND/ODER ZUR UNTERSTÜTZUNG VON SPORTGERÄTEN**
APPARATUS FOR STABILIZING BODY JOINTS AND/OR SUPPORTING ITEMS OF SPORTS EQUIPMENT
DISPOSITIF DE STABILISATION D'ARTICULATIONS DU CORPS ET/OU DE SOUTIEN D'APPAREILS DE SPORT

(30) Priorität: 07.12.2018 DE 102018131457
(43) Veröffentlichungstag der Anmeldung: 13.10.2021
(73) Patentinhaber: Betterguards Technology GmbH, 10625 Berlin (DE)
(72) Erfinder: BICHLER, Vinzenz, 10777 Berlin (DE); STUMPER, Timo, 12101 Berlin (DE); BUSCHINGER, Oscar, 10707 Berlin (DE)
(74) Vertreter: Okoampah, Rene
(86) Internationale Anmeldenummer: PCT/EP2019/083856
(87) Internationale Veröffentlichungsnummer: WO 2020/115227

(56) Entgegenhaltungen:
- EP-A1- 2 842 527
- EP-A1- 3 238 670
- WO-A1-2015/177357

## Beschreibung

### Technisches Gebiet

Die vorliegende Erfindung betrifft eine Vorrichtung zur Stabilisierung von Körpergelenken oder zur Unterstützung von Sportgeräten, welche eine Aufnahme, die mit einem Füllmedium gefüllt ist, einen ersten Körper zum Interagieren mit dem Füllmedium, wobei der erste Körper in der Aufnahme verschiebbar angeordnet ist, und ein Mittel zum Übertragen einer äußeren Kraft auf den ersten Körper, umfasst.

### Stand der Technik

Es ist bekannt Körpergelenke, Muskeln und Sehnen mittels Vorrichtungen, welche eine adaptive Bewegungsbegrenzung ermöglichen zu stabilisieren. Ferner ist es bekannt Sportgeräte die Ruckartigen Bewegungen ausgesetzt werden können, mit adaptiven Bewegungsbegrenzungsvorrichtungen zu versehen.

Unter anderem wird das adaptive Verhalten solcher Vorrichtungen dadurch erreicht, dass sich zwei Körper relativ zueinander bewegen, wobei sich zwischen den Körpern ein Füllmedium befindet. Dabei kann ein Körper der Vorrichtung eine Aufnahme bilden, die mit dem Füllmedium gefüllt ist. Der andere Körper kann einen Auszugskörper bilden, welcher in der Aufnahme bewegbar angeordnet ist. In dem Bereich zwischen der Aufnahme und dem Auszugskörper kann das Füllmedium strömen, wenn sich die beiden Körper relativ zueinander Bewegen. Die Fließgeschwindigkeit des Füllmediums hängt entscheidend von der Querschnittfläche senkrecht zu einer relativen Verschieberichtung der Aufnahme und des Auszugskörpers ab. Diese zur Strömung für das Füllmedium bereitstehende Querschnittfläche wird auch als hydraulischer Durchmesser bezeichnet und ist letztendlich für das reaktive Verhalten der Vorrichtung bei äußerer Krafteinwirkung entscheidend. So kann durch die Wahl des hydraulischen Durchmessers der Widerstand festgelegt werden, den die Vorrichtung äußeren Kräften entgegen bringt. Die Vorrichtungen können zwischen zwei Körperstellen eines Anwenders oder zwei sich relativ zueinander bewegbaren Elementen eines Sportgeräts fixiert werden.

Werden über die beiden Körperstellen des Anwenders physiologische Kräfte, das heißt für das entsprechend zu stabilisierende Körperteil oder Bauteil unkritische Kräfte, in die Vorrichtung eingeleitet, so wird gemäß dem hydraulischen Durchmessers in der Vorrichtung eine entsprechende Relativbewegung der Aufnahme und des Auszugskörpers und damit eine Bewegung des zu stabilisierenden Körperteils zugelassen.

Werden hingegen unphysiologische Kräfte, das heißt für das entsprechend zu stabilisierende Körperteil oder Bauteil kritische Kräfte, in die Vorrichtung eingeleitet, ist aufgrund des hydraulischen Durchmessers eine Relativbewegung zwischen dem Auszugskörper und der Aufnahme nur noch unter sehr hohem Kraftaufwand möglich.

Aus der EP 3 238 670 A1, die den nächstliegenden Stand der Technik darstellt, ist eine Vorrichtung zur Stabilisierung von Körpeergelenken bekannt, die ein adaptives Verhalten in Abhängigkeit der Intensität einer einwirkenden Kraft ermöglicht.

Die Vorrichtung umfasst eine Aufnahme, die mit einem Füllmedium gefüllt ist, einen ersten Körper zum Interagieren mit dem Füllmedium, wobei der erste Körper in der Aufnahme verschiebbar angeordnet ist, und ein Kraftübertragungsmittel zum Übertragen einer äußeren Kraft auf den ersten Körper. Ein zweiter Körper zum Interagieren mit dem Füllmedium ist in der Aufnahme verschiebbar angeordnet, wobei der zweite Körper über ein Kopplungselement elastisch mit dem ersten Körper gekoppelt ist. Ferner weist der zweite Körper eine Durchlassöffnung auf, durch welche das Füllmedium strömen kann. Die Durchlassöffnung in dem zweiten Körper stellt einen hydraulischen Durchmesser für das Füllmedium bereit, durch welchen das Füllmedium fließen kann, solange zwischen dem ersten Körper und dem zweiten Körper ein Abstand besteht.

Der erste Körper bildet dabei einen Ventilkörper und der zweite Körper einen Ventilsitz, so dass ein Fluss des Mediums durch die Durchlassöffnung in Abhängigkeit von der Ventilstellung zugelassen oder unterbunden werden kann.

Auf den ersten Körper einwirkende äußere Kräfte können über das Kopplungselement auf den zweiten Körper zu übertragen werden. Entsprechend ist der erste Körper dazu in der Lage, den zweiten Körper mittels des Kopplungselements durch das Füllmedium zu schieben und/oder zu ziehen.

Dabei ist das Kopplungselement derart konfiguriert, dass es beim Einwirken einer äußeren Kraft auf den ersten Körper, im Bereich einer physiologischen Geschwindigkeit, eine Kraft auf den zweiten Körper überträgt, so dass dieser gemeinsam mit dem ersten Körper durch das Füllmedium bewegt werden kann.

Führt die über den ersten Körper und das Kopplungselement auf den zweiten Körper wirkende Kraft zu kritischen relativen Verschiebegeschwindigkeiten in der Vorrichtung, das heißt zu unphysiologischen Geschwindigkeiten, gibt das Kopplungselement nach, wodurch sich der erste Körper auf den zweiten Körper zubewegt. Dadurch verringert sich der hydraulische Durchmesser, bis das durch die beiden Körper gebildete Ventil geschlossen ist.

Wenn kein hydraulischer Durchmesser mehr zur Verfügung steht, durch den das Füllmedium fließen kann, können der erste Körper und der zweite Körper nicht weiter durch das Füllmedium bewegt werden. Die Vorrichtung blockiert.

Es hat sich gezeigt, dass ein Bedarf für vergleichsweise noch kürzere Reaktionszeiten bis zum vollständigen Blockieren einer Bewegung, besteht, bei welchem die vorstehend beschriebene Vorrichtung an ihre Grenzen gelangt.

Ferner hat sich gezeigt, dass mit der vorstehend beschriebenen Vorrichtung ein Schließen der Durchlassöffnung nicht immer einwandfrei garantiert werden kann. Dies kann zu Unregelmäßigkeiten des Verhaltens der Vorrichtung führen.

### Darstellung der Erfindung

Ausgehend von dem bekannten Stand der Technik ist es eine Aufgabe der vorliegenden Erfindung, eine verbesserte Vorrichtung zur Stabilisierung von Körpergelenken oder zur Unterstützung von Sportgeräten bereitzustellen.

Die Aufgabe wird durch eine Vorrichtung zum zur Stabilisierung von Körpergelenken oder zur Unterstützung von Sportgeräten mit den Merkmalen des Anspruchs 1 gelöst. Vorteilhafte Weiterbildungen ergeben sich aus den Unteransprüchen.

Entsprechend wird eine Vorrichtung zum zur Stabilisierung von Körpergelenken oder zur Unterstützung von Sportgeräten vorgeschlagen, umfassend eine Aufnahme wobei die Aufnahme mit einem Füllmedium gefüllt ist, einen ersten Körper zum Interagieren mit dem Füllmedium, wobei der erste Körper in der Aufnahme verschiebbar angeordnet ist, einen Kraftübertragungskörper zum Übertragen einer äußeren Kraft auf den ersten Körper, einen zweiten Körper zum Interagieren mit dem Füllmedium, welcher in der Aufnahme verschiebbar angeordnet ist, wobei der zweite Körper über ein Kopplungselement elastisch mit dem ersten Körper gekoppelt ist, wobei der zweite Körper und/oder der erste Körper mindestens eine Durchlassöffnung aufweist, durch welche das Füllmedium strömen kann, und wobei der erste Körper einen Ventilkörper bildet und der zweite Körper einen Ventilsitz bildet, so das ein Fluss des Füllmediums durch die Durchlassöffnung in Abhängigkeit von der Ventilstellung zugelassen oder unterbunden werden kann. Erfindungsgemäß überlappt der zweite Körper in einer relativen Verschieberichtung zum ersten Körper mit diesem.

Eine Überlappung des ersten Körpers und des zweiten Körpers in einer relativen Verschieberichtung ermöglicht es die beiden Körper zu führen, wenn sie aufgrund einer äußeren Krafteinwirkung relativ zueinander bewegt werden. Dadurch kann die Wahrscheinlichkeit erhöht werden, dass ein kontaktieren der beiden Körper, welches das blockieren der Durchlassöffnung zur Folge hat, die Durchlassöffnung vollständig geschlossen ist. Dies trägt zu einem gleichmäßigen Verhalten der Vorrichtung bei.

Die relative Verschieberichtung bezeichnet die Richtung, in der der erste Körper und der zweite Körper durch eine äußere Krafteinwirkung und/oder das Kopplungselement relativ zueinander bewegt werden können.

In einer bevorzugten Weiterbildung umfasst der zweite Körper mindestens einen Vorsprung in der relativen Verschieberichtung, wobei der Vorsprung mit dem ersten Körper in Bezug auf die relative Verschieberichtung unabhängig von einer relativen Verschiebung des ersten Körpers und des zweiten Körpers zu einander überlappt.

Dadurch ist es möglich stets einen Kontakt, d. h. eine Führung des ersten Körpers am 2. Körper bereitzustellen. Folglich besteht eine Überlappung des Vorsprung des zweiten Körpers mit dem ersten Körper sowohl wenn sich die Vorrichtung in einer Ausgangsposition befindet, in welcher keine äußere Kraft auf die Vorrichtung und insbesondere den 1. und den zweiten Körper wirkt, als auch in einer Blockierposition, in welcher der erste Körper auf dem zweiten Körper aufsitzt und dabei die Durchlassöffnung verschließt.

In einer weiter bevorzugten Ausführungsform weist der zweite Körper mindestens eine sich in die relative Verschieberichtung erstreckenden Oberfläche auf, um den ersten Körper in der relativen Verschieberichtung zu führen. Die Führungsoberfläche kann an dem ersten Körper anliegen und diesen entlang eines vollständigen Verschiebehubs, d. h. zwischen der Ausgangsposition und der blockiert Position der Vorrichtung, kontaktieren.

In einer Weiterbildung weist der erste Körper und/oder der zweite Körper gegenüber dem jeweils anderen Körper mindestens eine Hinterschneidung auf, um einen relativen Verschiebeweg zwischen den beiden Körpern zu begrenzen. Der relative Verschiebeweg zwischen dem ersten Körper und dem zweiten Körper hat unmittelbar Einfluss auf die Reaktionszeit der Vorrichtung. Durch die Wahl eines geeigneten relativen Verschiebewegs wird neben dem Kopplungselement eine weitere Einstellungsmöglichkeit der Reaktionszeit der Vorrichtung bereitgestellt.

Eine Hinterschneidung trägt dazu bei, dass der erste Körper und der zweite Körper sich nicht nur überlappen, sondern zumindest teilweise hintergreifen. Die Hinterschneidung der beiden Körper kann in unterschiedlicher Form bereitgestellt werden. Beispielsweise kann eine Hinterschneidung an einem in Bezug auf die relative Verschieberichtung äußersten Punkt eines überlappenden Abschnitts des zweiten Körpers angeordnet sein. Alternativ können sowohl der erste Körper als auch der zweite Körper jeweils eine Hinterschneidung aufweisen, die eine Ausgangsposition des ersten Körpers und des zweiten Körpers in der Ausgangsposition der Vorrichtung definieren.

In einer bevorzugten Weiterbildung definiert der Vorsprung des zweiten Körpers einen Aufnahmeraum, in dem der erste Körper zumindest teilweise aufgenommen ist, wobei der Aufnahmeraum einen relativen Verschiebeweg des ersten Körpers und des zweiten Körpers zueinander in der relativen Verschieberichtung begrenzt. Der Aufnahmeraum erlaubt es sowohl eine geführte Relativbewegung zwischen dem ersten Körper und dem zweiten Körper als auch eine Begrenzung des maximalen relativen Verschiebewegs zwischen dem ersten Körper und dem zweiten Körper bereitzustellen.

Der Aufnahmeraum kann eine oder mehrere Öffnungen aufweisen. So kann der erste Körper beispielsweise teilweise aus dem Aufnahmeraum herausragen. In letzterem Fall ist nur ein Teil des ersten Körpers ständig in dem Aufnahmeraum aufgenommen, während ein anderer Teil des ersten Körpers in Abhängigkeit einer relativen Verschiebeposition der beiden Körper zueinander aus dem Aufnahmeraum herausragt. Dadurch kann eine Führung des ersten Körpers und des zweiten Körpers zueinander bereitgestellt werden.

In einer weiter bevorzugten Ausführungsform umfasst der Vorsprung einen oder mehrere Ausnehmungen, die mit der Durchlassöffnung in Fluid Verbindung stehen, um in einer offenen Ventilstellung einen Fluss des Füllmediums relativ zum zweiten Körper, bevorzugt durch den zweiten Körper, zu ermöglichen. Dadurch kann die Möglichkeit des Füllmediums innerhalb der Aufnahme relativ zu dem ersten Körper und insbesondere dem zweiten Körper zu fließen, so lange aufrechterhalten werden, bis der erste Körper die Durchlassöffnung des zweiten Körpers blockiert.

Die mindestens eine Ausnehmung kann auch eine Montagefunktion erfüllen. So kann die Ausnehmung derart gestaltet sein, dass der erste Körper durch die Ausnehmung in den Aufnahmeraum des zweiten Körpers einsetzbar ist.

Gemäß einer weiter bevorzugten Weiterbildung unterteilen der zweite Körper und/oder der erste Körper eine Kavität der Aufnahme in eine erste Kammer und eine zweite Kammer. Bewegen sich der erste Körper und der zweite Körper aufgrund der Einwirkung einer äußeren Kraft bei geöffneter Ventilstellung durch die Aufnahme, so kann das Füllmedium durch die Durchlassöffnung des zweiten Körpers von der ersten Kammer in die zweite Kammer strömen.

In einer weiter bevorzugten Ausgestaltung ist das Kopplungselement in einer Ausgangsposition der Vorrichtung zwischen dem ersten Körper und dem zweiten Körper vorgespannt, wobei der erste Körper mit dem zweiten Körper in Kontakt steht.

In den gemäß dem Stand der Technik bekannten Vorrichtungen, die ohne Vorspannung des Kopplungselements - zum Beispiel einer Feder - ausgeführt sind, führen Kopplungselemente mit vergleichsweise hohen Widerstandseigenschaften, wie zum Beispiel einer hohen Federstärke, zu vergleichsweise langen schließ wegen. Letztere können sich negativ auf die Reproduzierbarkeit des Verhaltens der Vorrichtung auswirken.

Der Mangel einer Vorspannung des Kopplungselements führt auch dazu, dass der erste Körper bereits beim Einwirken von vergleichsweise geringen Kräften auf den zweiten Körper zu bewegt wird, wodurch auch der potentielle Fließweg des Wirkmediums hin zu der Durchlassöffnung bereits reduziert wird. Dies kann zu einem ungenauen, nicht reproduzierbaren Verhalten der Vorrichtung bei dem Einwirken vergleichsweise geringfügiger äußerer Kräfte auf den 1. und/oder den zweiten Körper führen.

Durch die Vorspannung des Kopplungselements können Kopplungselement mit geringeren Widerstandskräften eingesetzt werden. Die Vorspannung des Kopplungselements kann dann so gewählt werden, dass das Kopplungselement beim Einwirken von vergleichsweise geringen äußeren Kräften noch nicht reagiert und eine Relativbewegung zwischen dem 1. und dem zweiten Körper ausbleibt. Insgesamt lässt sich durch die Vorspannung des Kopplungselements eine Reaktionsschwelle in Bezug auf die auf die Vorrichtung einwirkenden äußeren Kräfte heraufsetzen.

Darüber hinaus können durch die Vorspannung des Kopplungselements kürzere relative Verschiebewege zwischen dem ersten Körper und dem zweiten Körper realisiert werden. Mit anderen Worten kann der maximale Hub zwischen dem ersten Körper und dem zweiten Körper durch eine Vorspannung des Kopplungselements reduziert werden. Durch einen kürzeren maximalen relativen Verschiebeweg zwischen dem ersten Körper und dem zweiten Körper kann der Reaktionsweg und somit die Reaktionszeit bis zum Aufbau einer ausreichend hohen Widerstandskraft der Vorrichtung signifikant verringert werden. Schließlich hat die Vorspannung des Kopplungselements auch Einfluss auf die Bauteilabmessung der Vorrichtung, so kann die Gesamtlänge der Vorrichtung um die Verkürzung der relativen Verschiebewege reduziert werden.

### Kurze Beschreibung der Figuren

Bevorzugte weitere Ausführungsformen der Erfindung werden durch die nachfolgende Beschreibung der Figuren näher erläutert. Dabei zeigen:
- Fig. 1A: schematisch eine perspektivische Ansicht einer Vorrichtung zur Stabilisierung von Körpergelenken und/oder Sportgeräten,
- Fig. 1B: schematisch eine seitliche Schnittansicht der Vorrichtung aus Fig. 1A in einem Ausgangszustand,
- Fig. 2A: schematisch eine Detailansicht der Vorrichtung gemäß Fig. 1B in einer Ausgangsposition,
- Fig. 2B: schematisch eine Detailansicht der Vorrichtung gemäß Fig. 1B in einer Blockierposition,
- Fig. 3: schematisch eine perspektivische Detailansicht eines zweiten Körpers,
- Fig. 4A: schematisch eine seitliche Detailansicht des ersten und zweiten Körpers,
- Fig. 4B: schematisch eine Rückansicht des ersten und zweiten Körpers gemäß Fig. 4A,
- Fig. 5A: schematisch eine Detailansicht einer alternativen Ausführungsform des ersten und zweiten Körpers,
- Fig. 5B: schematisch eine Detailansicht einer alternativen Ausführungsform des ersten und zweiten Körpers,
- Fig. 6: schematisch eine Detailansicht einer alternativen Ausführungsform des ersten und zweiten Körpers,

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

Die Funktion der vorliegenden Vorrichtung wird nachstehend anhand des Einsatzes im Sportbereich beschrieben. Dabei kommt die Vorrichtung zur Dämpfung der Bewegung zweier Punkte eines Körpers, die sich relativ zueinander bewegen können, zum Einsatz. Ein solcher Körper kann beispielsweise ein Sportschuh sein, der gekoppelt mit der vorliegenden Vorrichtung einer Umknickbewegung entgegenwirken kann. Die vorliegende Vorrichtung ist jedoch nicht auf die hierin beschriebenen Einsatzgebiete beschränkt. So kann sie auch zwischen zwei Körperteilen eines Lebewesens angeordnet werden, um eine entsprechende Körperbewegung zu dämpfen. Alternativ kann die Vorrichtung auch in anderen Alltagsgegenständen verwendet werden, in welchen abrupt ansteigende Kräfte zwischen eines Körpers oder eines Gegenstands gedämpft werden sollen.

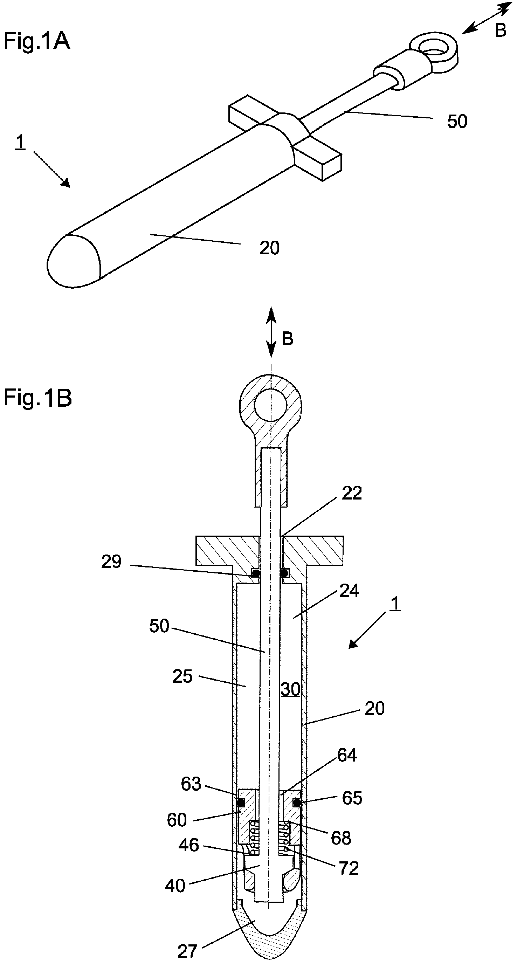

**Fig. 1A** ist eine perspektivische Ansicht einer Vorrichtung 1 zur Stabilisierung von Körpergelenken und/oder Sportgeräten zu entnehmen. Aus einer zylindrischen Aufnahme 20 ragt ein Kraftübertragungsmittel 50 heraus. Dabei kann die Aufnahme 20 an einem Körperpunkt eines Anwenders oder Sportgeräts wie zum Beispiel eines Sportschuhs befestigt werden. Das Ende des Kraftübertragungsmittels 50, welches außerhalb der Vorrichtung 20 liegt, kann an einem zweiten Körperpunkt befestigt werden. Der erste Körperpunkt und der zweite Körperpunkt zeichnen sich dadurch aus, dass sie abrupten Relativbewegungen zueinander ausgesetzt sind. Die Richtung B stellt die Bewegungsrichtung der Vorrichtung dar. Alternative kann die Aufnahme auch quaderförmig ausgebildet sein.

**Fig. 1B** zeigt eine Schnittansicht der Vorrichtung 1, welche sich in einem Ausgangszustand befindet. Die Aufnahme 20 weist eine Öffnung 22 auf, durch welche ein Kraftübertragungsmittel 50 in den Innenraum der Vorrichtung 20 hineinragt. Bewegt sich der Körperpunkt, an welchem die Aufnahme 20 befestigt ist, relativ zu dem Körperpunkt, an dem das Kraftübertragungsmittel 50 angeordnet ist, so bewegt sich das Kraftübertragungsmittel 50 relativ zu der Aufnahme 20. Insbesondere kann sich das Kraftübertragungsmittel 50 in einer Bewegungsrichtung B weiter in die Aufnahme 20 hinein oder weiter aus der Aufnahme 20 heraus bewegen.

Die Aufnahme 20 der Vorrichtung 1 ist aus Edelstahl gefertigt. Alternativ kann die Aufnahme auch aus Kunststoff gefertigt sein. Unter anderem könne auch Faserverstärkte Kunststoffe zum Einsatz kommen. Alternativ kann die Aufnahme auch aus anderen Metallen wie zum Beispiel Aluminium oder Magnesium gefertigt sein. Darüber hinaus kann die Aufnahme auch aus Keramik gefertigt sein. Das Kraftübertragungsmittel 50 ist durch ein Kabel aus Draht gebildet und erstreckt sich von einen ersten Körper 40 durch eine Durchlassöffnung 64 eines zweiten Körpers 60 und schließlich durch die Öffnung 22 der Aufnahme 20. Alternativ kann das Kraftübertragungsmittel 50 in Form eines Stabelements aus Kunststoff ausgebildet sein. Ferner kann das Kraftübertragungsmittel auch faserförmig ausgebildet sein. Des Weiteren kann das Kraftübertragungsmittel auch aus Metall, wie zum Beispiel Aluminium, Magnesium oder Edelstahl, gefertigt sein.

Ein Innenraum 24 der Vorrichtung 20 ist mit einem Füllmedium 30 gefüllt. Bei dem Füllmedium 30 handelt es sich um ein newtonsches Fluid wie zum Beispiel Silikonöl. Alternativ können auch dilatante Fluide als Füllmedium verwendet werden. Darüber hinaus kann auch ein scherverdickender Kunststoff eingesetzt werden. Dabei liegt der Kunststoff in Pulverform vor. Des Weiteren kann auch Sand als Füllmedium zum Einsatz kommen.

Darüber hinaus ist im Innenraum 24 der Vorrichtung 20 ein erster Körper 40 angeordnet, welcher in der Bewegungsrichtung B relativ zur Aufnahme 20 durch das Füllmedium 30 bewegbar ist. In der vorliegenden Ausführungsform ist der erste Körper 40 mit dem Kraftübertragungsmittel 50 einstückig verbunden. Alternativ kann der erste Körper an einem Kraftübertragungsbereich an das Kraftübertragungsmittel gekoppelt sein, so dass eine von dem Kraftübertragungsmittel ausgehende Kraft auf den ersten Körper übertragen werden kann.

Der Querschnitt des ersten Körpers 40 ist stets kleiner als der Querschnitt der Aufnahme 20, so dass das Füllmedium 30 in Bewegungsrichtung B relativ zu dem ersten Körper 40 fließen kann. Der Abstand zwischen dem ersten Körper und der Aufnahme 20 bildet einen hydraulischen Durchmesser.

Der erste Körper 40 ist aus Kunststoff gefertigt. Alternativ kann der erste Körper auch aus einem Metall wie zum Beispiel Aluminium, Magnesium oder Stahl gefertigt sein.

Des Weiteren ist im Innenraum 24 der Aufnahme 20 ein zweiter Körper 60 angeordnet, welcher relativ zur Aufnahme 20 in der Bewegungsrichtung B bewegbar ist..

Der zweite Körper 60 ist aus Kunststoff gefertigt. Alternativ kann der zweite Körper auch aus einem Metall wie zum Beispiel Aluminium gefertigt sein. Der zweite Körper weist eine umlaufende Nut 63 zur Aufnahme eines O-Rings 65 auf. Der zweite Körper 60 unterteilt den Innenraum 24 der Aufnahme 20 in eine erste Kammer 25 und eine zweite Kammer 27. Der O-Ring 65 verhindert, dass das Füllmedium 30 entlang einer äußeren Umfangsoberfläche des zweiten Körpers 60 zwischen der ersten Kammer 25 und der zweiten Kammer 27 strömen kann.

Der erste Körper 40 ist mit dem zweiten Körper 60 über ein elastisches Kopplungselement 70 gekoppelt. Das in Fig. 1B gezeigte elastische Kopplungselement ist durch eine Feder 72 gebildet, welche an einem Ende in einem Federsitz 46 des ersten Körpers 40 und am anderen Ende in einem Federsitz 68 des zweiten Körpers 60 gelagert ist. Der zweite Körper 60 kann über die Feder 72 mittels des ersten Körpers 40 geschoben werden. Dabei kann die Feder 72 aus Kunststoff oder aus Metall gefertigt sein. Alternativ kann das elastische Kopplungselement auch in Form eines elastischen Polymers oder Kautschuks ausgebildet sein.

In einer weiteren Alternative sind der erste Körper, der zweite Körper und das elastische Kopplungselement einstückig spritzgegossen.

Ferner umfasst der zweite Körper 60 eine Durchlassöffnung 64, durch welche das Füllmedium 30 strömen kann. Wird der zweite Körper 60 entsprechend von einer, von dem Kopplungselement ausgehenden Kraft relativ zur Aufnahme 20 bewegt, kann das Füllmedium 30 durch die Durchlassöffnung 64 des zweiten Körpers 60 zwischen der ersten Kammer 25 und der zweiten Kammer 27 strömen. Entsprechend definiert die Durchlassöffnung 64 einen hydraulischen Durchmesser für das Füllmedium 30 im Bereich des zweiten Körpers 60.

Die in den Fig. 1A und 1B gezeigte Vorrichtung 1 ist auf Zugbelastungen ausgelegt. D.h., auf Belastungen, welche aus einem Auseinanderbewegen des Körperpunkts, an dem die Aufnahme 20 befestigt ist und des Körperpunkts, an dem das Kraftübertragungsmittel 50 befestigt ist, resultieren. Wird das Kraftübertragungsmittel 50 aus der Vorrichtung 20 herausgezogen, so zieht es den ersten Körper 40 mit sich, wodurch dieser mittels des Kopplungselements 70 auf den zweiten Körper 60 drückt. Das Kraftübertragungsmittel 50 erstreckt sich von dem ersten Körper 40 durch die Durchlassöffnung 64 des zweiten Körpers 60 und schließlich durch die Öffnung 22 der Aufnahme 20. Im Bereich der Öffnung 22 sind Dichteinsätze 29 angeordnet, welche den Innenraum 24 der Aufnahme 20 gegenüber der Umgebung abdichten, so dass das Füllmedium 30 im Innenraum 24 der Aufnahme 20 gehalten werden kann.

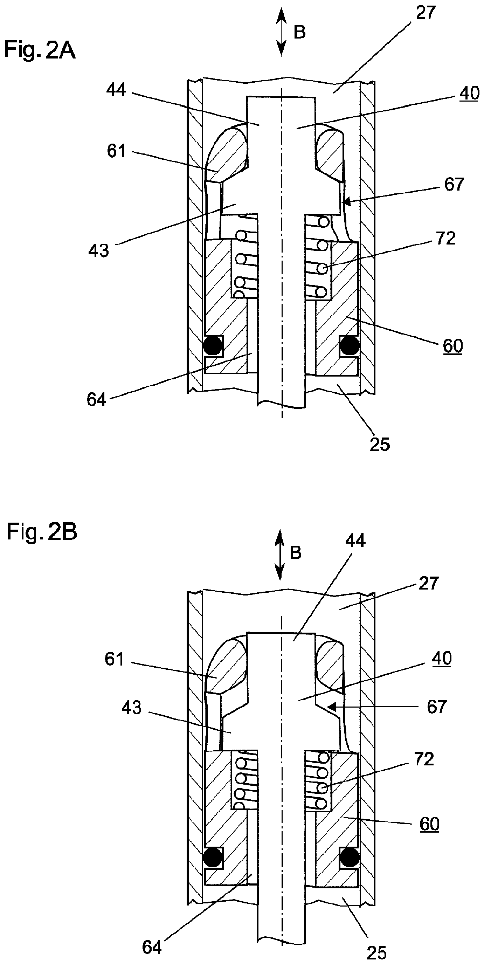

**Figuren 2A** und **2B** zeigen Detailansichten der Vorrichtung anhand welcher die Interaktion des ersten Körpers 40 mit dem zweiten Körper 60 näher beschrieben wird. Der zweite Körper 60 weist einen Vorsprung 61 auf, der sich hin zu dem ersten Körper 40 erstreckt und mit diesem überlappt. Der Vorsprung 61 ist dazu konfiguriert, den ersten Körper 40 zu hintergreifen. Dabei definiert der Vorsprung 61 einen Aufnahmeraum 67, in dem der erste Körper 40 teilweise aufgenommen ist.

Gemäß der vorliegenden Ausführungsform ist der erste Körper 40 stufenförmig aufgebaut. So weist der erste Körper 40 einen ersten Abschnitt 43 auf, der in dem Aufnahmeraum 67 des zweiten Körpers 60 verschiebbar aufgenommen ist und einen gegenüber dem ersten Abschnitt 43 im Durchmesser verjüngten zweiten Abschnitt 44, der sich aus dem Aufnahmeraum heraus in Richtung der Bewegungsrichtung B erstreckt. Der Vorsprung 61 ist derart konfiguriert, dass er den ersten Abschnitt 43 des ersten Körpers 40 hintergreift und eine Führung in Bewegungsrichtung B für den zweiten Abschnitt 44 bildet. Auf diese Weise lässt sich der erste Körper gegenüber dem zweiten Körper zentrieren, sodass stets ein Verschließen der Durchlassöffnung 64 sichergestellt werden kann, wenn der erste Körper 40 und der zweite Körper 60 eine geschlossene Ventilstellung einnehmen.

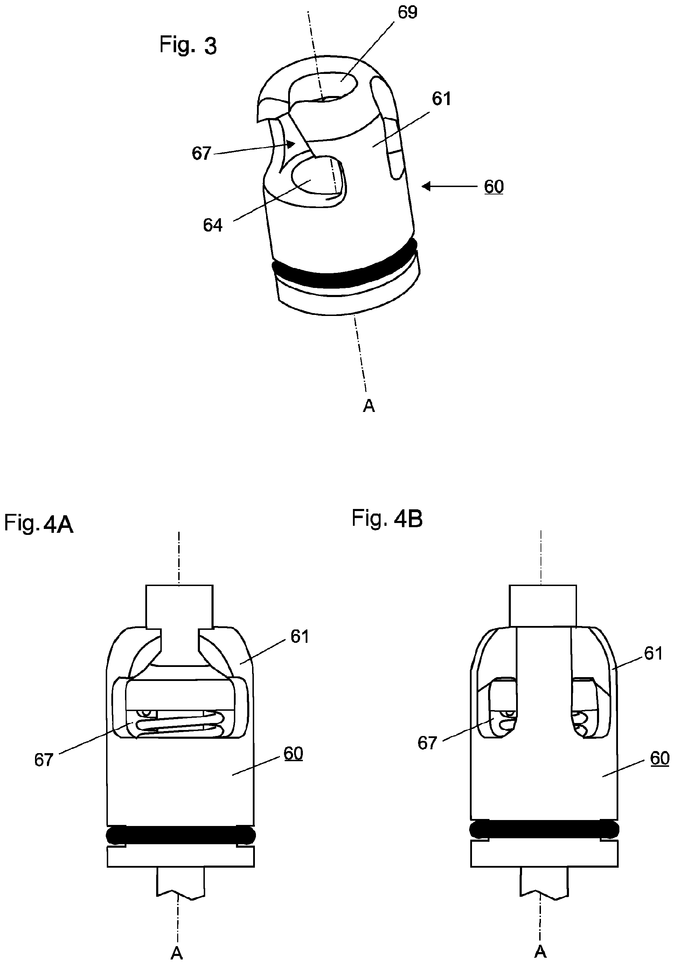

**Figur 3** zeigt eine perspektivische Ansicht des zweiten Körpers 60. Der Vorsprung 61 setzt sich in einer axialen Richtung A des zweiten Körpers 60 fort. An einem Endbereich des Vorsprung 61 ist dieser nach innen, hin zur Achse gekrümmt, wodurch eine Hinterschneidung zum hintergreifen des ersten Abschnitts des ersten Körpers gebildet ist. Der Vorsprung 61 definiert einen Aufnahmeraum 67, zur Aufnahme des ersten Abschnitts des ersten Körpers. Der Aufnahmeraum 67 grenzt an die Durchlassöffnung 64 an. An dem der Durchlassöffnung 64 gegenüberliegenden Ende des Aufnahmeraums 67 definiert die Hinterschneidung des Vorsprung 61 eine Öffnung, die als Führung 69 für den zweiten Abschnitt des ersten Körpers dient. Der zweiten Abschnitt des ersten Körpers kann in der relativen Verschieberichtung des ersten Körpers und des zweiten Körpers zueinander teilweise aus/in den Aufnahmeraum 67 heraus/hinein treten.

In Bezug auf die Längsachse A des zweiten Körpers 60 weist der Vorsprung 61 mehrere seitliche Unterbrechungen auf, wie auch den **Figuren 4A** und **4B** zu entnehmen ist. Diese erfüllen 2 Aufgaben. Zum einen ermöglicht eine seitliche Unterbrechung das Einsetzen des ersten Körpers in den Aufnahmeraum 67 des zweiten Körpers 60. Der Vorsprung 61 ist entsprechend flexibel gestaltet, sodass der erste Körper in den Aufnahmeraum 67 eingeklipst werden kann. Zum anderen, wie den **Figur 2A** und **2B** entnommen werden kann, gewähren die Unterbrechungen Fließwege für das Füllmedium 30 zwischen der zweiten Kammer 27 und der Durchlassöffnung 64.

Figur 2A zeigt die Ausgangsposition der Vorrichtung zum Dämpfen einer Bewegung. Der erste Abschnitt 43 des ersten Körpers 40 steht mit der Hinterschneidung des Vorsprungs 61 des zweiten Körpers 60 in Kontakt. Diese Ausgangsposition kann als offene Ventilstellung der Vorrichtung bezeichnet werden.

Die Feder 72 unterliegt in der in Figur 2A gezeigten Ausgangsposition der Vorrichtung 1 einer Vorspannung. Die Vorspannung der Feder 72 ist so gewählt, dass die Feder 72 beim Einwirken von vergleichsweise geringen äußeren Kräften noch nicht reagiert und eine Relativbewegung zwischen dem ersten Körper 40 und dem zweiten Körper 60 ausbleibt. Insgesamt ermöglicht die Vorspannung der Feder 72 eine höhere Reaktionsschwelle in Bezug auf die, auf die Vorrichtung 1 einwirkenden äußeren Kräfte.

Darüber hinaus können durch die Vorspannung der Feder kürzere relative Verschiebewege zwischen dem ersten Körper 40 und dem zweiten Körper 60 realisiert werden. Mit anderen Worten kann der maximale Hub zwischen dem ersten Körper 40 und dem zweiten Körper 60 durch eine Vorspannung der Feder 72 reduziert werden. Durch einen kürzeren maximalen relativen Verschiebeweg zwischen dem ersten Körper 40 und dem zweiten Körper 60 kann der Reaktionsweg und somit die Reaktionszeit bis zum Aufbau einer ausreichend hohen Widerstandskraft der Vorrichtung 1 signifikant verringert werden.

Nachstehend wird die Funktion der Vorrichtung anhand der **Figuren 2A und 2B** beschrieben. Wirkt eine Kraft im Bereich einer physiologischen Geschwindigkeit auf das Kraftübertragungsmittel 50, so dass der erste Körper 40 in Richtung des zweiten Körpers 60 gezogen wird, wird auch der zweite Körper 60 mittels der Feder 72 mitbewegt. Die Interaktion zwischen dem zweiten Körper 60 und dem Füllmedium 30 führt zu einer auf den zweiten Körper 60 wirkende Widerstandskraft. Die Widerstandskraft wird von dem 2. Auszugskörper 60 auf die Feder 72 übertragen. Würde die Feder 72 keiner Vorspannung unterliegen, so würde sie unmittelbar mit einer Einfederbewegung beginnen. Im vorliegenden Fall unterliegt die Feder 72 jedoch einer Vorspannung, welche auf die Anordnung des ersten Körpers 40 in dem Aufnahmeraum 67 des zweiten Körpers 60 zurückzuführen ist. Mit anderen Worten hält der Vorsprung 61 des zweiten Körpers 60 den ersten Körper 40 gegenüber dem zweiten Körper 60 in einer vorgespannten Ausgangsposition.

Die Federstärke der Feder 72 ist derart gewählt, dass die vorgespannte Feder 72 erst beim Erreichen eines Schwellwerts, der auf den zweiten Körper 60 wirkenden Widerstandskraft, einfedert. Bei einer Widerstandskraft unterhalb des Schwellwerts findet keine Relativbewegung zwischen dem ersten Körper und dem zweiten Körper statt. Bei einer Widerstandskraft oberhalb des Schwellwerts beginnt die Feder einzufedern, wodurch sich der erste Körper 40 auf den zweiten Körper zu bewegt, bis die Durchlassöffnung 64 vollständig geschlossen ist, wie in Figur 2B gezeigt. In diesem Zustand beträgt der hydraulische Durchmesser der Vorrichtung Null. Somit ist kein hydraulischer Durchmesser mehr vorhanden, durch den das Füllmedium 30 zwischen der ersten Kammer 25 und der zweiten Kammer 27 strömen kann.

Durch die Wahl von vergleichsweise geringen Feder stärken lässt sich in Kombination mit der Vorspannung der Feder eine vergleichsweise schnelle Reaktionszeit der Vorrichtung, d. h. ein schnelles Schließen der Durchlassöffnung 64 realisieren. Ein strömen des Füllmediums von der ersten Kammer in die zweite Kammer ist nicht länger möglich, sodass die beiden Auszugskörper 40, 60 nicht länger relativ zur Aufnahme 20 bewegt werden können. In diesem Zustand lässt die Vorrichtung 1 keine weitere Bewegung zwischen zwei zu unterstützenden/dämpfenden Körperpunkten zu.

Beispielsweise kann der Schwellwert 4,5 N betragen und das System bei einem Sprung einer Widerstandskraft von 4,5 N auf 5 N sofort vollständig schließen.

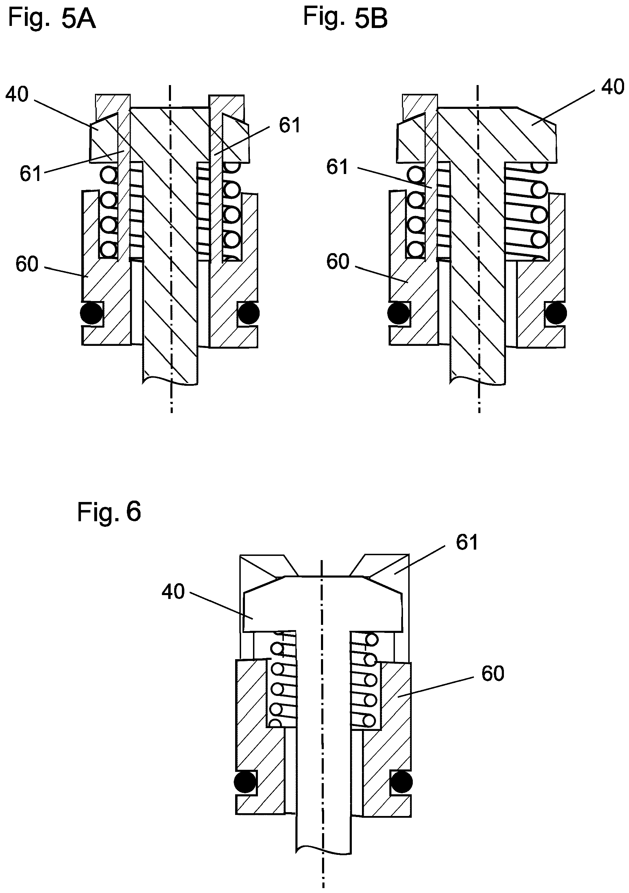

Figuren 5A bis 7B zeigen alternative Ausführungsformen des ersten und zweiten Körpers. Figur 5A zeigt einen zweiten Körper 60 mit Vorsprüngen 61, die durch den ersten Körper 40 hindurch führen. Am Ende der Vorsprünge 61 sind Hinterschneidungen angeordnet, beispielsweise in Form von Flanschen, die ein Hintergreifen des ersten Körpers 40 ermöglichen.

Figur 5B unterscheidet sich dadurch von Figur 5A, dass der zweite Körper 60 nur einen Vorsprung 61 aufweist, der durch den ersten Körper 40 hindurch geführt ist, um diesen zu hinter greifen.

Figur 6 zeigt einen zweiten Körper 60 mit hakenförmigen Vorsprüngen 61, die dazu eingerichtet sind, den ersten Körper 40 zu hintergreifen. Die Vorsprünge 61 führen dabei seitlich an dem ersten Körper 40 vorbei.

Das Funktionsprinzip der Ausführungsformen gemäß Figuren 5A bis 6 gleicht dem vorstehend beschriebenen Funktionsprinzip gemäß Figuren 2A und 2B.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 1: Vorrichtung

- 20: Aufnahme
- 22: Öffnung
- 24: Innenraum
- 25: Erste Kammer
- 26: Innenfläche
- 27: Zweite Kammer
- 28: Stufe
- 29: Dichteinsatz

- 30: Füllmedium

- 40: Erster Körper
- 43: Erster Abschnitt
- 44: Zweiter Abschnitt
- 46: Federsitz

- 50: Kraftübertragungsmittel

- 60: Zweiter Körper
- 61: Vorsprung
- 62: Ausnehmung
- 63: Nut
- 64: Durchlassöffnung
- 65: O-Ring
- 66: Führungsvorsprung
- 67: Aufnahmeraum
- 68: Federsitz
- 69: Führung

- 70: Kopplungselement
- 72: Feder
- A: Axiale Richtung
- B: Bewegungsrichtung

## Patentansprüche

1. Vorrichtung (1) zur Stabilisierung von Körpergelenken und/oder zur Unterstützung von Sportgeräten, umfassend:
eine Aufnahme (20), wobei die Aufnahme (20) mit einem Füllmedium (30) gefüllt ist,
einen ersten Körper (40) zum Interagieren mit dem Füllmedium (30), wobei der erste Körper in der Aufnahme (20) verschiebbar angeordnet ist,
einen Kraftübertragungskörper (50) zum Übertragen einer äußeren Kraft auf den ersten Körper (40),
einen zweiten Körper (60) zum Interagieren mit dem Füllmedium (30), welcher in der Aufnahme (20) verschiebbar angeordnet ist,
wobei der zweite Körper über ein Kopplungselement (70) elastisch mit dem ersten Körper (40) gekoppelt ist,
wobei der zweite Körper (60) und/oder der erste Körper (40) mindestens eine Durchlassöffnung (64) aufweist, durch welche das Füllmedium (30) strömen kann,
und
wobei der erste Körper (40) einen Ventilkörper bildet und der zweite Körper (60) einen Ventilsitz bildet, so das ein Fluss des Füllmediums (30) durch die Durchlassöffnung (64) in Abhängigkeit von der Ventilstellung zugelassen oder unterbunden werden kann,
**dadurch gekennzeichnet, dass**
der zweite Körper in einer relativen Verschieberichtung zum ersten Körper mit diesem überlappt.

2. Vorrichtung (1) gemäß Anspruch 1, **dadurch gekennzeichnet, dass** der zweite Körper mindestens einen Vorsprung in der relativen Verschieberichtung umfasst, wobei der Vorsprung mit dem ersten Körper in Bezug auf die relative Verschieberichtung unabhängig von einer relativen Verschiebung des ersten Körpers und des zweiten Körpers zueinander überlappt.

3. Vorrichtung (1) gemäß Anspruch 1 oder 2, **dadurch gekennzeichnet, dass** der erste Körper und/oder der zweite Körper gegenüber dem jeweils anderen Körper mindestens eine Hinterschneidung aufweist, um den relativen Verschiebeweg zwischen den beiden Körpern zu begrenzen.

4. Vorrichtung (1) gemäß einem der vorhergehenden Ansprüche, **dadurch gekennzeichnet, dass** der zweite Körper mindestens eine sich in der relativen Verschieberichtung ersteckende Führungsoberfläche aufweist, um den ersten Körper in der relativen Verschieberichtung zu führen.

5. Vorrichtung (1) gemäß einem der vorhergehenden Ansprüche, **dadurch gekennzeichnet, dass** der Vorsprung des ersten Körpers einen Aufnahmeraum definiert, in dem der erste Körper zumindest teilweise aufgenommen ist, wobei der Aufnahmeraum einen relativen Verschiebeweg des ersten Körpers und des zweiten Körpers zueinander in der relativen Verschieberichtung begrenzt.

6. Vorrichtung (1) gemäß einem der vorhergehenden Ansprüche, **dadurch gekennzeichnet, dass** der Vorsprung eine Unterbrechung oder mehrere Unterbrechungen umfasst, die mit der Durchlassöffnung (64) in Fluidverbindung stehen, um in einer offenen Ventilstellung einen Fluss des Füllmediums (30) relativ zum zweiten Körper, bevorzugt durch den zweiten Körper, zu ermöglichen.

7. Vorrichtung (1) gemäß einem der vorhergehenden Ansprüche, **dadurch gekennzeichnet, dass** der zweite Körper und/oder der erste Körper eine Kavität der Aufnahme (20) in eine erste Kammer und eine zweite Kammer unterteilt.

8. Vorrichtung (1) gemäß Anspruch 7, **dadurch gekennzeichnet, dass** die Durchlassöffnung (64) in einer offenen Ventilstellung einen Strom des Fluidmediums (30) zwischen der ersten Kammer und der zweiten Kammer bereitstellen kann.

9. Vorrichtung (1) gemäß einem der vorhergehenden Ansprüche, **dadurch gekennzeichnet, dass** das Kopplungselement (70) in einer Ausgangsposition der Vorrichtung zwischen dem ersten Körper und dem zweiten Körper vorgespannt ist, wobei der erste Körper mit dem zweiten Körper in Kontakt steht.

## Claims

1. A device (1) for stabilizing body joints and/or for supporting items of sports equipment, said device comprising:
a receptacle (20), wherein the receptacle (20) is filled with a filling medium (30),
a first body (40) for interacting with the filling medium (30), wherein the first body is arranged displaceably in the receptacle (20),
a force transferring body (50) for transferring an external force to the first body (40),
a second body (60) for interacting with the filling medium (30), which is arranged displaceably in the receptacle (20),
wherein the second body is elastically coupled to the first body (40) by way of a coupling element (70),
wherein the second body (60) and/or the first body (40) have at least one passage opening (64), through which the filling medium (30) can flow,
and
wherein the first body (40) forms a valve body and the second body (60) forms a valve seat, with the result that a flow of the filling medium (30) through the passage opening (64) can be permitted or prevented depending on the valve position,
**characterized in that**
the second body overlaps with the first body in a relative displacement direction to the first body.

2. The device (1) as claimed in claim 1, **characterized in that** the second body comprises at least one projection in the relative displacement direction, wherein the projection overlaps the first body with respect to the relative displacement direction independently of a relative displacement of the first body and of the second body with respect to one another.

3. The device (1) as claimed in claim 1 or 2, **characterized in that** the first body and/or the second body has at least one undercut with respect to the respective other body, in order to delimit the relative displacement distance between the two bodies.

4. The device (1) as claimed in one of the preceding claims, **characterized in that** the second body has at least one guide surface extending in the relative displacement direction, in order to guide the first body in the relative displacement direction.

5. The device (1) as claimed in one of the preceding claims, **characterized in that** the projection of the first body defines a receiving space, in which the first body is at least partially received, wherein the receiving space delimits a relative displacement distance of the first body and the second body in relation to one another in the relative displacement direction.

6. The device (1) as claimed in one of the preceding claims, **characterized in that** the projection comprises an interruption or a plurality of interruptions, which are fluidically connected to the passage opening (64), in order to allow a flow of the filling medium (30) relative to the second body, preferably through the second body, in an open valve position.

7. The device (1) as claimed in one of the preceding claims, **characterized in that** the second body and/or the first body subdivide a cavity of the receptacle (20) into a first chamber and a second chamber.

8. The device (1) as claimed in claim 7, **characterized in that** the passage opening (64) can provide a flow of the fluid medium (30) between the first chamber and the second chamber in an open valve position.

9. The device (1) as claimed in one of the preceding claims, **characterized in that** the coupling element (70) is preloaded between the first body and the second body in a starting position of the device, wherein the first body is in contact with the second body.

## Revendications

1. Dispositif (1) pour la stabilisation d'articulations de corps et/ou pour le soutien d'appareils de sport, comprenant :
un logement (20), dans lequel le logement (20) est rempli d'un milieu de remplissage (30), un premier corps (40) pour l'interaction avec le milieu de remplissage (30), dans lequel le premier corps est agencé de manière déplaçable dans le logement (20),
un corps de transmission de force (50) pour la transmission d'une force extérieure au premier corps (40),
un second corps (60) pour l'interaction avec le milieu de remplissage (30), qui est agencé de manière déplaçable dans le logement (20),
dans lequel le second corps est couplé par le biais d'un élément de couplage (70) élastiquement au premier corps (40),
dans lequel le second corps (60) et/ou le premier corps (40) présente au moins une ouverture de passage (64), par laquelle le milieu de remplissage (30) peut s'écouler, et
dans lequel le premier corps (40) forme un corps de soupape et le second corps (60) forme un siège de soupape de sorte qu'un flux du milieu de remplissage (30) par l'ouverture de passage (64) puisse être autorisé ou empêché en fonction de la position de soupape,
**caractérisé en ce que**
le second corps chevauche le premier corps dans un sens de déplacement relatif par rapport à celui-ci.

2. Dispositif (1) selon la revendication 1, **caractérisé en ce que** le second corps comporte au moins une saillie dans le sens de déplacement relatif, dans lequel la saillie chevauche le premier corps par rapport au sens de déplacement relatif indépendamment d'un déplacement relatif du premier corps et du second corps l'un par rapport à l'autre.

3. Dispositif (1) selon la revendication 1 ou 2, **caractérisé en ce que** le premier corps et/ou le second corps présente par rapport à l'autre corps respectivement au moins une contre-dépouille afin de limiter la course de déplacement relatif entre les deux corps.

4. Dispositif (1) selon l'une quelconque des revendications précédentes, **caractérisé en ce que** le second corps présente au moins une surface de guidage s'étendant dans le sens de déplacement relatif afin de guider le premier corps dans le sens de déplacement relatif.

5. Dispositif (1) selon l'une quelconque des revendications précédentes, **caractérisé en ce que** la saillie du premier corps définit un espace de réception, dans lequel le premier corps est reçu au moins partiellement, dans lequel l'espace de réception limite une course de déplacement relatif du premier corps et du second corps l'un par rapport à l'autre dans le sens de déplacement relatif.

6. Dispositif (1) selon l'une quelconque des revendications précédentes, **caractérisé en ce que** la saillie comporte une interruption ou plusieurs interruptions qui sont en liaison fluidique avec l'ouverture de passage (64) afin de permettre dans une position de soupape ouverte un flux du milieu de remplissage (30) par rapport au second corps, de préférence par le second corps.

7. Dispositif (1) selon l'une quelconque des revendications précédentes, **caractérisé en ce que** le second corps et/ou le premier corps divise une cavité du logement (20) en une première chambre et une seconde chambre.

8. Dispositif (1) selon la revendication 7, **caractérisé en ce que** l'ouverture de passage (64) peut fournir dans une position de soupape ouverte un courant du milieu de fluide (30) entre la première chambre et la seconde chambre.

9. Dispositif (1) selon l'une quelconque des revendications précédentes, **caractérisé en ce que** l'élément de couplage (70) est précontraint dans une position de départ du dispositif entre le premier corps et le second corps, dans lequel le premier corps est en contact avec le second corps.
